# EUROPEAN PATENT APPLICATION

(11) **EP 3 501 652 A1**
(43) Date of publication of application: **26.06.2019**
(21) Application number: 18181431.0
(22) Date of filing: 03.07.2018
(51) Int. Cl.: B01L 3/00, G01N 33/557, G01N 21/84

(54) **TESTING APPARATUS AND CONTROL METHOD THEREOF**

(30) Priority: 22.12.2017 KR 20170178570
(71) Applicant: Samsung Electronics Co., Ltd., Suwon-si, Gyeonggi-do (KR)
(72) Inventor: Kim, Seung Hyun, Anyang-si, Gyeonggi-do (KR); Hwang, Kyu Youn, Sejong-si (KR)
(74) Representative: Gulde & Partner

(57) **Abstract**

A testing apparatus and control method thereof are provided. The testing apparatus includes a detector configured to measure optical characteristic values on a control line and a test line formed on a reactor; and a controller configured to obtain an optical characteristic value measured, by the detector, on the control line, determine a reaction time corresponding to the optical characteristic value measured on the control line based on predetermined reaction time information, and determine a measurement time at which the detector is to measure another optical characteristic value on the test line based on the reaction time that is determined

## Description

### BACKGROUND

### 1. Field

The disclosure relates to a testing apparatus and control method thereof capable of performing in vitro testing with a reduced amount of sample.

### 2. Discussion of Related Art

In Vitro Diagnostics (IVD) may be performed through immunity tests, clinical chemistry tests, etc., using patient samples, and plays an important role in diagnosis and treatment of disease and determination of convalescence.

IVD may be performed by identifying the presence of a particular target substance in a sample, or the concentration of the particular target substance in the sample. To perform IVD, a reaction between a reagent and the sample may be used, and a color reaction, in particular, between a target substance and a reagent may be used to identify the presence of the target substance or the concentration of the target substance in the sample.

### SUMMARY OF THE INVENTION

The disclosure provides a testing apparatus and control method thereof, which may improve reliability and accuracy of test results by estimating the elapsed reaction time based on a degree of color development on a control line of a reactor in which a reaction between a sample and a reagent occurs, and determining a measurement time at which to measure a degree of color development on a test line of the reactor based on the estimated elapsed reaction time.

The disclosure also provides a testing apparatus and control method thereof, which may improve reliability and accuracy of test results by determining that an error has occurred, and by informing the user that the error has occurred in association with a flow of a sample based on the saturation value of an optical characteristic value measured on a control line varying from a predetermined reference value.

In accordance with an aspect of the disclosure, there is provided a testing apparatus comprising: a detector configured to measure optical characteristic values on a control line and a test line formed on a reactor; and a controller configured to obtain an optical characteristic value measured, by the detector, on the control line, determine a reaction time corresponding to the optical characteristic value measured on the control line based on predetermined reaction time information, and determine a measurement time at which the detector is to measure another optical characteristic value on the test line based on the reaction time.

The reaction time comprises an elapsed reaction time or a remaining reaction time.

The predetermined reaction time information comprises information that matches predetermined optical characteristic values with respective reaction times.

The controller is further configured to determine the measurement time based on the elapsed reaction time and an expected reaction completion time based on the reaction time comprising the elapsed reaction time.

The controller is configured to determine the measurement time based on the remaining reaction time and a current time based on the reaction time comprising the remaining reaction time.

The testing apparatus further comprises a storage configured to store the predetermined reaction time information when the reaction time information is obtained by the testing apparatus using information associated with the reactor.

The testing apparatus further comprises a display configured to display the reaction time.

The controller is further configured to compare a saturation value of the optical characteristic value measured on the control line with a predetermined saturation value and determine whether an error has occurred based on comparing the saturation value and the predetermined saturation value.

The controller is further configured to output information identifying that the error has occurred based on the saturation value of the optical characteristic value measured on the control line being less than the predetermined saturation value.

The controller is further configured to determine that the optical characteristic value measured on the control line is saturated based on a change in the optical characteristic value measured on the control line within a predetermined period of time being less than a predetermined reference change.

In accordance with an aspect of the disclosure, there is provided a control method of a testing apparatus, the control method comprising: obtaining, by the testing apparatus, predetermined reaction time information for a control line; measuring, by the testing apparatus, an optical characteristic value on the control line; determining, by the testing apparatus, a reaction time corresponding to the optical characteristic value measured on the control line based on the predetermined reaction time information; and determining, by the testing apparatus, a measurement time at which the testing apparatus is to measure another optical characteristic value on a test line based on the reaction time.

The reaction time comprises an elapsed reaction time or a remaining reaction time.

The predetermined reaction time information comprises information that maps predetermined optical characteristic values with respective reaction times.

The determining of the measurement time comprises determining the measurement time based on the elapsed reaction time and an expected reaction completion time based on the reaction time comprising the elapsed reaction time.

The determining of the measurement time comprises determining the measurement time based on the remaining reaction time and a current time based on the reaction time comprising the remaining reaction time.

The control method further comprises storing the predetermined reaction time information when the predetermined reaction time information is obtained.

The control method further comprises displaying the reaction time.

The control method further comprises comparing a saturation value of the optical characteristic value measured on the control line with a predetermined saturation value and determining whether an error has occurred based on comparing the saturation value and the predetermined saturation value.

The determining of whether the error has occurred comprises determining that the error has occurred when the saturation value of the optical characteristic value measured on the control line is less than the predetermined saturation value.

The control method further comprises determining that the optical characteristic value measured on the control line is saturated based on a change in the optical characteristic value measured on the control line within a predetermined period of time being less than a predetermined reference change.

In accordance with an aspect of the disclosure, there is provided a device comprising a memory configured to store instructions; and at least one processor configured to execute the instructions to: obtain a measured optical characteristic value of a substance formed on a control line of a reactor, determine a reaction time corresponding to the measured optical characteristic value of the substance formed on the control line of the reactor based on the measured optical characteristic value and predetermined reaction time information, determine a measurement time at which to measure another optical characteristic value of another substance formed on a test line of the reactor based on the reaction time, and measure the other optical characteristic value of the other substance based on the measurement time.

The predetermined reaction time information includes information that maps known optical characteristic values of the substance and known reaction times associated with a reaction that forms the substance.

The at least one processor is further configured to: determine an elapsed reaction time based on the reaction time; identify an expected reaction completion time; and determine the measurement time based on the elapsed reaction time and the expected reaction completion time.

The at least one processor is further configured to: determine a remaining reaction time based on the reaction time; identify a current time; and determine the measurement time based on the remaining reaction time and the current time.

The substance is formed based on a sample that is applied to the reactor and a substance formed on the control line of the reactor.

The at least one processor is further configured to: determine a measured saturation value based on the measured optical characteristic value; compare the measured saturation value and a predetermined saturation value; and determine whether an error has occurred based on comparing the measured saturation value and the predetermined saturation value.

The substance is formed based on a biological fluid that is applied to the reactor.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a control block diagram of a testing apparatus, according to an embodiment;
FIG. 2 is a perspective view illustrating the exterior of a testing apparatus, according to an embodiment;
FIG. 3 shows the structure of a detection area included on a reactor;
FIGS. 4, 5, and 6 show a principle of detecting a target substance to be analyzed by chromatography;
FIGS. 7, 8, 9, and 10 show the structure of a detection area including a conjugate pad and the detection principle;
FIG. 11 show exemplary test results from color development on a control line and a test line;
FIG. 12 is an exemplary graph representing optical characteristic values on a control line according to elapsed reaction time;
FIG. 13 shows an exemplary table of remaining reaction times matched with respective optical characteristic values on a control line;
FIGS. 14, 15, and 16 show exemplary screens displayed on a display of a testing apparatus, according to an embodiment;
FIG. 17 is an exemplary graph representing saturation values of optical characteristic values on a control line;
FIG. 18 shows an exemplary warning screen displayed when a flow error occurs on a reactor;
FIG. 19 is a flowchart illustrating a control method of a testing apparatus, according to an embodiment; and
FIG. 20 is a flowchart illustrating a procedure of detecting a flow error in a control method of the testing apparatus according to an embodiment.

### DETAILED DESCRIPTION

Like numerals may refer to like elements throughout the specification.

It should be understood that the term "connect" or its derivatives may refer both to direct and indirect connection (e.g., wired or wireless connections), and that indirect connection includes a connection over a wireless communication network.

The terms "include (or including)" and "comprise (or comprising)" are inclusive or openended and do not exclude additional unrecited elements or method steps, unless otherwise mentioned.

It should be understood that the singular forms "a," "an," and "the" include plural references unless the context clearly dictates otherwise.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

Furthermore, the terms, such as "part", "block", "member", "module", etc., may refer to a unit configured to perform at least one function or operation. For example, the terms may refer to a unit implemented in hardware, firmware, or a combination of hardware and software. The terms may refer to a unit including a processor (e.g., a central processing unit (CPU), a graphics processing unit (GPU), an accelerated processing unit (APU), etc.), a microprocessor, and/or any processing component (e.g., a field-programmable gate array (FPGA), an application-specific integrated circuit (ASIC), etc.) that interprets and/or executes instructions. The terms may refer to a unit including one or more processors capable of being programmed to perform a function.

Embodiments of a testing apparatus and control method of the same will now be described with reference to the accompanying drawings.

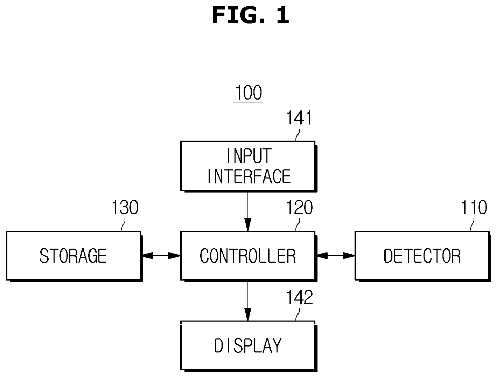

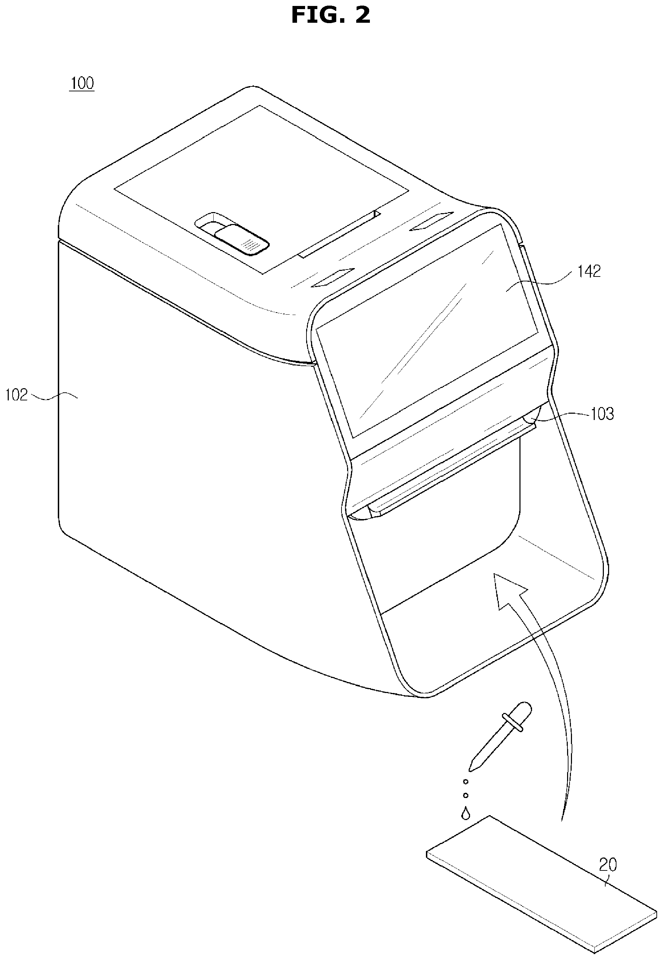

FIG. 1 is a control block diagram of a testing apparatus, according to an embodiment, and FIG. 2 is a perspective view illustrating the exterior of a testing apparatus, according to an embodiment.

As shown in FIG. 1, a testing apparatus 100 includes a detector 110 for measuring an optical characteristic value of a substance formed based on a sample being applied on a particular area of a reactor 20, a controller 120 for determining the presence of a target substance in the sample or the concentration of the target substance in the sample based on the measured optical characteristic value of the substance, a storage 130 for storing information required for the controller 120 to determine the presence of a target substance or the concentration of the target substance, a display 142 for displaying test results, and an input interface 141 for receiving test-related commands from a user.

The optical characteristic values measured by the detector 110 may correspond to various values, such as optical density (OD) values indicating a degree to which a substance absorbs light, reflectivity values indicating a degree to which a substance reflects light, transmittance values indicating a degree to which light passes through a substance, and/or the like.

The detector 110 may include a camera, an optical sensor, and/or the like. For example, when the detector 110 includes the optical sensor, the detector 110 may include a light source for generating and irradiating light, and a receiver for receiving light that has been transmitted through or reflected from the reactor 20. When the detector 110 measures reflected light, the light source and the receiver are located in the same direction with respect to the reactor 20, and when the detector 110 measures transmitted light, the light source and the receiver may be located in opposite directions with respect to the reactor 20.

The light source may include a light emitting diode (LED), or may be implemented with any other light source of a different type, such as a semiconductor laser, a helium-neon (He-Ne) laser, a halogen lamp, etc. The light source may further include an additional device, such as a filter for irradiating a particular wavelength of light.

The receiver may detect light that has been transmitted through or reflected from the reactor 20, and convert the detected light to an electric signal corresponding to the light intensity. The receiver 112 may include a light receiving device such as a photo diode.

The controller 120 may determine the presence of a target substance or the concentration of the target substance based on an optical characteristic value output from the detector 110. For example, determination of the presence of a target substance may be made by comparing a predetermined reference value and the measured optical characteristic value. The concentration of the target substance may be calculated by applying the measured optical characteristic value to a pre-stored calibration curve. The calibration curve may represent a relationship between optical characteristic values and concentrations of a target substance. A method for estimating the concentration based on the measured optical characteristic value may use an end point-based method, a kinetic-based method, and/or the like.

Furthermore, the controller 120 may determine and control a measurement time for the detector 110 to measure the optical characteristic value in association with a particular area of the reactor 20, as described elsewhere herein.

The controller 120 may include at least one memory for storing software (e.g., a program) containing one or more instructions for carrying out the aforementioned and following operations, and at least one processor for executing the one or more instructions. The controller 120 may perform one or more processes described herein based on executing software instructions stored by a computer-readable medium, such as memory and/or storage 130. A computer-readable medium is defined herein as a non-transitory memory device.

The storage 130 may include non-volatile and/or volatile memories and may appropriately distribute information divided by the type and use to the non-volatile and/or volatile memories. The controller 120 and the storage 130 may share memory. When executed, software instructions stored in the storage 130 may cause the controller 120 to perform one or more processes described herein.

The display 142 may be implemented with one of various types of display devices, such as an LED display, an organic LED (OLED) display, a liquid crystal display (LCD), a plasma display panel (PDP), a cathode ray tube (CRT), etc.

The input interface 141 may be implemented with a keyboard, a keypad, a switch, a touch panel, and/or the like. For example, a touch screen may be implemented by a touch panel mounted on the front surface of the display 142, and a power button may be implemented by a physical key. In this case, once the testing apparatus 100 is powered on, the user may enter a control command by contacting the touch screen.

As shown in FIG. 2, a main body 102 that forms the exterior of the testing apparatus 100 is equipped with a door 103, and the user may open the door 103 to insert the reactor 20 into the main body 102.

The user may insert the reactor 20 into the main body 102 after applying (e.g., injecting, dropping, etc.) a sample onto the reactor 20.

After the reactor 20 is inserted, the controller 120 of the testing apparatus 100 may measure an optical characteristic value of a substance formed on a particular area of the reactor 20 and determine the presence of a target substance or the concentration of the target substance based on the measured optical characteristic value, in a predetermined sequence.

Although FIG. 2 depicts the reactor 20 as being inserted into the testing apparatus 100, other embodiments may include the reactor 20 being inserted into the testing apparatus 100 in the form of being mounted on another device, such as a disc-type platform.

The sample to be applied to the reactor 20 may be a biological sample such as blood, tissue fluid, lymph fluid, urine, saliva, bone marrow fluid, and/or the like. The user may apply the sample onto the reactor 20 using a tool, such as a pipette, a syringe, a dropper, and/or the like.

The user may apply a sample, which is diluted by a buffer, onto the reactor 20, or may apply the sample onto the reactor 20 and then subsequently apply the buffer to the sample to dilute the sample.

The user may insert the reactor 20 into the testing apparatus 100 after applying the sample onto the reactor 20. In some situations, an amount of time may elapse between when the sample is applied onto the reactor 20 and when the reactor 20 is inserted to the testing apparatus 100. In such cases, if an optical characteristic value is measured without reflecting the amount of time that has elapsed, the test result may be inaccurate and thereby unreliable.

Accordingly, in an embodiment, the testing apparatus 100 may determine a measurement time at which to measure the optical characteristic value by taking into account a point in time when a reaction is actually started or when the sample is injected to the reactor 20, i.e., the amount of time that may have elapsed. In association with reactors 20 that use a chromatography technique, as will be described elsewhere herein, the sample may move rapidly by capillary force as soon as the sample is applied to the reactor 20. As such, the time at which the reaction is started and the time at which the sample is applied to the reactor 20 may be substantially concurrent. Operation of the testing apparatus 100 will now be described in detail in association with an exemplary reaction that occurs on the reactor 20.

The sample may react with a first labeled conjugate before being applied to the reactor 20. The first labeled conjugate may be a complex of a captor substance that singularly reacts with a target substance, and a labeled substance. For example, if the target substance is an antigen Q, then the first labeled conjugate may be a complex of a conjugate of an antibody Q that singularly reacts with the antigen Q, and a labeled substance.

The labeled substance may include metal colloids such as latex beads, gold colloids, silver colloids, etc., an enzyme such as peroxidase, a fluorescent substance, a luminescent substance, a super-paramagnetic substance, a substance having lanthanide chelate and a radioactive isotope, and/or the like.

Additionally, the sample may also react with a second labeled conjugate that binds to a second captor substance that is fixed to the control line of the reactor 20 to enhance reliability and/or accuracy of the reaction results. The second labeled conjugate is a conjugate of a substance that singularly reacts with the second captor substance, and a labeled substance. The labeled substance may be one of the labeled substances as enumerated above. As an example, if the second captor substance fixed to the control line is biotin, then the labeled conjugate may be a complex of a conjugate of streptavidin, and the labeled substance.

In this case, the antigen Q that is present in the sample may be applied to the reactor 20 with a flow of the sample while being bound to the first labeled conjugate having the antibody Q, in which case, the second labeled conjugate having streptavidin may also be applied to the reactor 20.

In this embodiment, it is assumed that the reactor 20 uses chromatography, that uses capillary force, to detect the presence of a target substance.

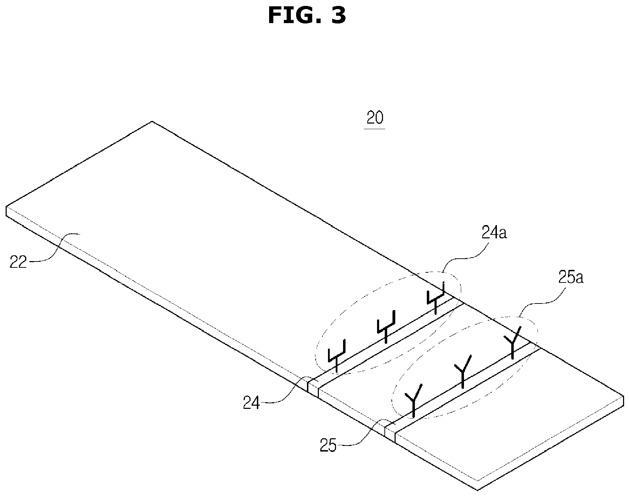

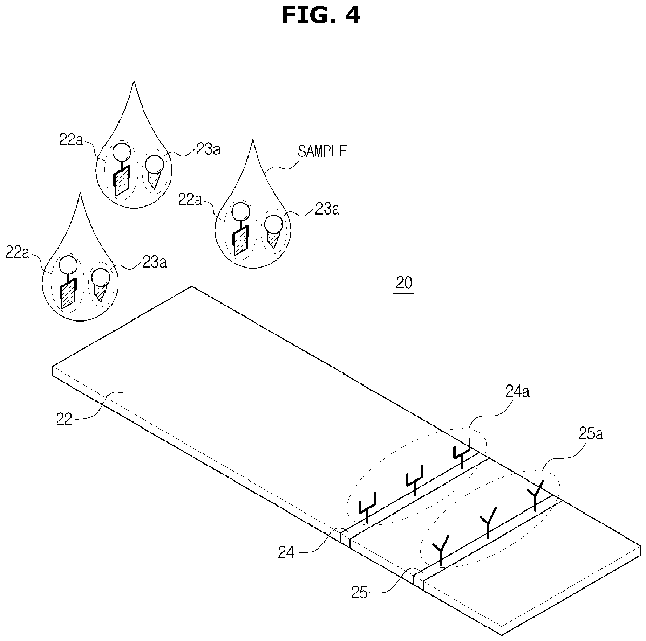

FIG. 3 shows the structure of a detection area included in the reactor 20, and FIGS. 4, 5, and 6 show a principle of detecting a target substance to be analyzed by chromatography.

The reactor 20 may include a porous thin membrane such as cellulose, micropores, micropillars, and/or the like, and may permit movement of the sample by capillary force. In some embodiments, the reactor 20 may colloquially be known as a strip, a test strip, and/or the like.

As shown in FIG. 3, the reactor 20 includes a set of areas formed at different locations on the reactor 20. For example, the reactor 20 includes a sample pad 22, to which a sample is applied, that is formed on one side of the reactor 20. As further shown in FIG. 3, the reactor 20 includes a test line 24, to which a first captor substance 24a is permanently fixed to detect a target substance, formed on another side of the reactor 20. As used herein, the term "permanently fixed" may refer to the first captor substance 24a being substantially fixed to the test line 24 so as to not be moved by a flow of the sample as the sample flows from the sample pad 22 towards and beyond the test line 24. As further shown in FIG. 3, the reactor 20 includes a control line 25 to which a second captor substance 25a is permanently fixed. As shown in FIG. 3, the test line 24 may be disposed between the sample pad 22 and the control line 25.

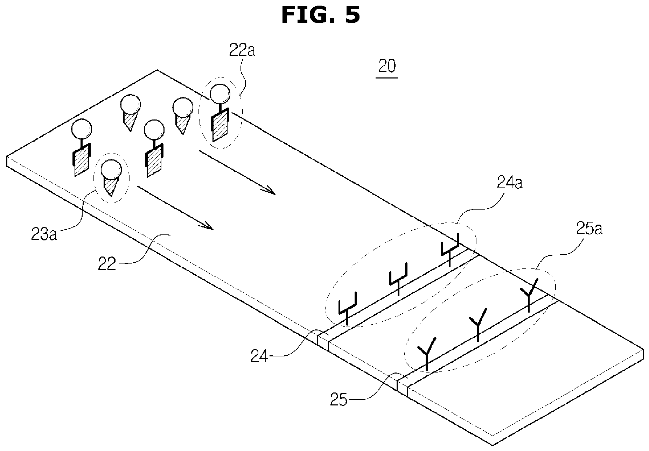

As shown in FIGS. 4 and 5, when a sample, such as blood or urine, is applied to the sample pad 22, the sample flows towards the test line 24 by capillary force. If the target substance is the antigen Q and a binding reaction of the target substance and the first labeled conjugate occurs, then the sample that reaches a detection area of the reactor 20 includes a complex 22a of the antigen Q and the first labeled conjugate.

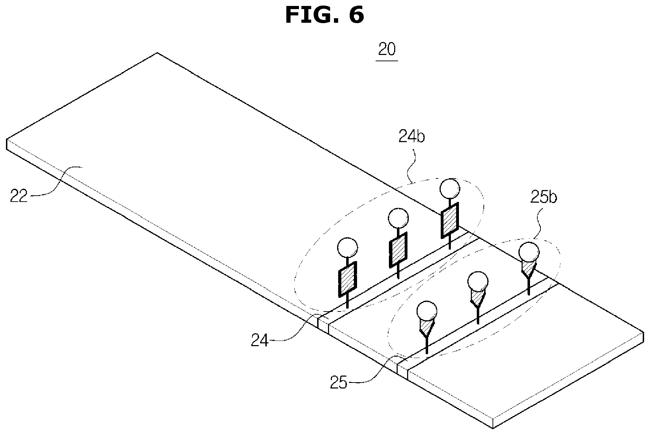

With the target substance, which is the antigen Q, the first captor substance 24a that is permanently fixed to the test line 24 may be the antibody Q. Accordingly, as shown in FIG. 6, when the sample flows by capillary force and reaches the test line 24, the complex 22a of the antigen Q and the first labeled conjugate is bound with the antibody Q (e.g., the first captor substance 24a), thereby forming a sandwich complex 24b. Therefore, when a target substance is present in the sample, the target substance may be detected by a labeled substance (e.g., sandwich complex 24b) on the test line 24.

Various factors, such as a small amount of sample, an abnormal flow of the sample, a contamination of the sample, and/or the like, may inhibit reliable and/or accurate testing. To identify the prevalence of one or more of the foregoing factors, some embodiments utilize the control line 25 including the second captor substance 25a. The second captor substance 25a may singularly react with a particular substance included in the sample regardless of whether the target substance is present in the sample.

As an example, biotin may be used as the second captor substance 25a that is permanently fixed to the control line 25. In such cases, the second labeled conjugate 23a that is present in the sample may be a streptavidin-labeled substance conjugate, which has an enhanced binding property in relation to biotin.

As shown in FIGS. 4 through 6, the second labeled conjugate 23a, having a particular substance that singularly reacts with the second captor substance 25a, is present in the sample that has been applied to the reactor 20. If the sample flows across reactor 20 from the sample pad 22 towards the test line 24 and/or the control line 25 by osmotic pressure, then the second labeled conjugate 23a is also moved with the movement of the sample. Accordingly, regardless of the presence of a target substance in the sample, and as shown in FIG. 6, the complex 25b of the second labeled conjugate 23a and the second captor substance 25a is formed and color development from the labeled substance (e.g., the complex 25b) appears on the control line 25.

As described above, the sample may be mixed and/or react with a labeled conjugate before being applied to the reactor 20. Alternatively, the labeled conjugate may be formed and temporarily fixed on a conjugate pad 23 on the reactor 20. As used herein, "temporarily fixed" may refer to the labeled conjugate being fixed to the conjugate pad 23 in such a way such that the labeled conjugate is capable of moving across the reactor 20 with the flow of the sample. In other words, as the flow of the sample moves across the conjugate pad 23, the flow of the sample may mix with the labeled conjugate to permit the labeled conjugate to move with the flow of the sample towards the test line 24 and/or the control line 25, as described below.

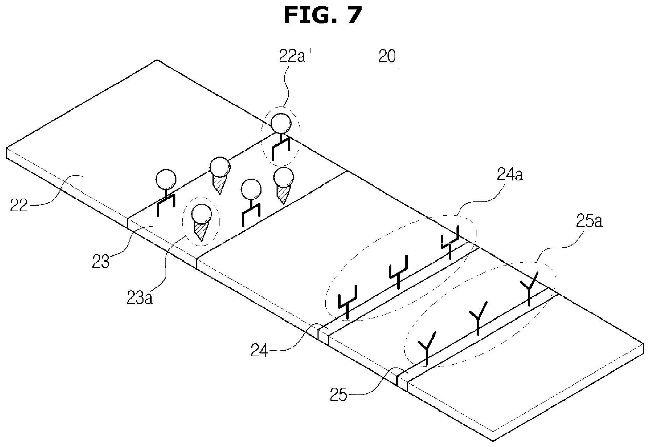

FIGS. 7 to 10 show the structure of a detection area including a conjugate pad and the detection principle.

As shown in FIG. 7, the reactor 20 may include the sample pad 22, the test line 24, the control line 25, and the conjugate pad 23. A first labeled conjugate 22a', which is a conjugate of a first captor substance that singularly reacts with a target substance and a labeled substance, may be temporarily fixed to the conjugate pad 23. In addition, a second labeled conjugate 23a, which is a conjugate of a material that singularly reacts with the second captor substance 25a fixed to the control line 25 and a labeled substance, may also be temporarily fixed to the conjugate pad 23.

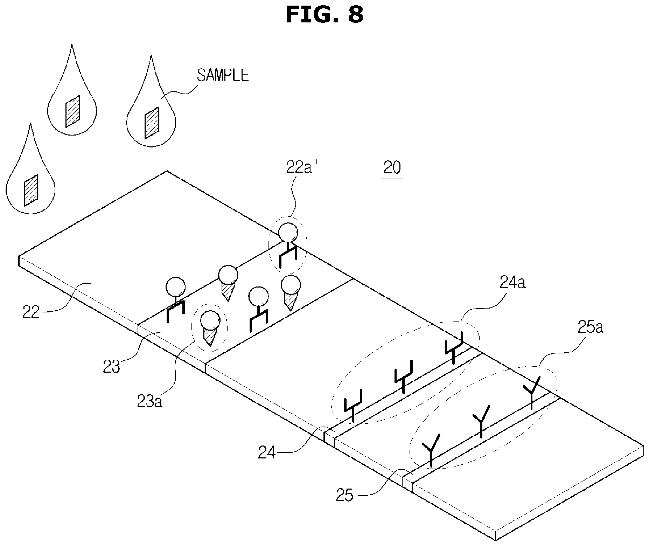

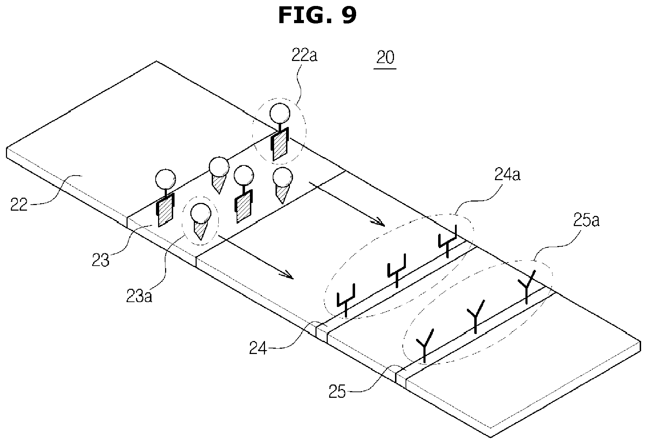

As shown in FIG. 8, when a sample, such as blood, is applied to the sample pad 22, the sample flows from the sample pad 22 towards the control line 25 by osmotic pressure. As shown in FIG. 9, and in situations where a target substance is included in the sample, the target substance and the first labeled conjugate 22a' bind together to form a target substance-labeled conjugate complex 22a. The sample continues to flow by osmotic pressure, and the flow of the sample carries the target substance-labeled conjugate complex 22a and the second labeled conjugate 23a towards the test line 24.

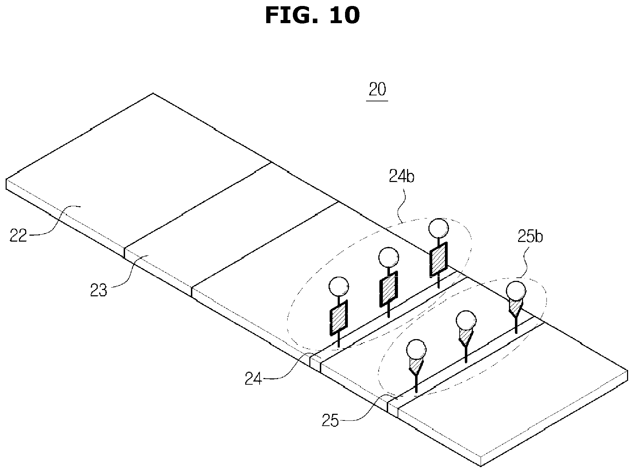

As shown in FIG. 10, and in situations where the flow of the sample reaches the test line 24 and/or the control line 25, the first captor substance 24a and the target substance-labeled conjugate complex 22a bind together to form the sandwich complex 24b on the test line 24. A complex 25b resulting from binding of the second labeled conjugate 23a and the second captor substance 25a is formed on the control line 25. In this way, the controller 120 may obtain an optical characteristic value of a substance formed on the reactor 20, as described below.

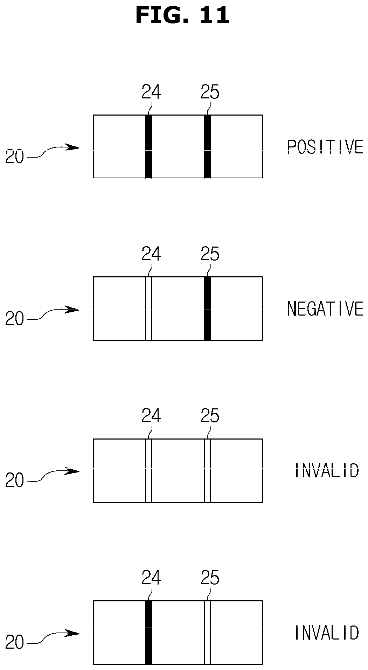

FIG. 11 shows exemplary test results from color development on a control line and a test line.

The controller 120 may control the detector 110 to measure degrees of color development of substances on the test line 24 and/or the control line 25 of the reactor 20. The degree of color development of the substances may be measured as an optical characteristic value, such as an OD value, an absorbance value, and/or the like. As used herein, a substance formed on the test line 24 and/or the control line 25 may refer to one or more substances associated with a sample. For example, a substance may include a substance formed based on a reaction between a substance included in a sample and a substance permanently fixed to a control line 25 and/or a test line 24.

The detector 110 sends the measured optical characteristic values to the controller 120, and the controller 120 determines the presence of a target substance in the sample based on the measured optical characteristic values. The controller 120 may also estimate the concentration of the target substance based on a calibration curve and the measured optical characteristic values.

As shown in FIG. 11, the controller 120 may determine that a target substance is present in the sample based on a color reaction occurring on both the test line 24 and the control line 25 (e.g., may make a positive determination). For example, the controller 120 may make the positive determination if the measured optical characteristic value of a substance on the control line 25 is equal to or greater than a predetermined first reference value, and the measured optical characteristic value of another substance on the test line 24 is equal to or greater than a predetermined second reference value. The first reference value for an optical characteristic value of the control line 25 and the second reference value for an optical characteristic value of the test line 24 may be different values.

As further shown in FIG. 11, the controller 120 may determine that the target substance is not present in the sample based on a color reaction occurring on the control line 25, and a color reaction not occurring on the test line 24 (e.g., may make a negative determination). For example, the controller 120 may make the negative determination if the measured optical characteristic value of the substance on the control line 25 is equal to or greater than the first reference value, and the measured optical characteristic value of the other substance on the test line 24 is less than the second reference value.

As further shown in FIG. 11, the controller 120 may determine that the results are invalid in the situation where color reactions fail to occur on both the test line 24 and the control line 25, and/or where a color reaction fails to occur on the control line 25. For example, the controller 120 may determine that the results are invalid if the measured optical characteristic value of the substance on the control line 25 is less than the first reference value. A color reaction may fail to occur on the control line 25 in the situation where the sample fails to adequately flow across the reactor 20. The controller 120 may detect this situation based on the measured optical characteristic value of the substance on the control line 25 being less than the first reference value.

The time at which color development appears may depend on the type of color reaction on the test line 24 and/or the control line 25. In some cases, color development may be designed to appear earlier on the control line 25 as compared to the test line 24.

In some cases, and as a certain time elapses after the reaction is started, a color reaction, unrelated to the existence of the target substance in the sample, may occur on the test line 24 due to a back flow effect of the sample. For example, the sample that has moved to the control line 25 may move back to the test line 24 and cause a singular color reaction. In such cases, the detector 110 may measure a degree of color development that appears on the test line 24 based on this effect, thereby reducing reliability and/or accuracy of the test results.

The controller 120 may improve reliability and/or accuracy of the test results by estimating a reaction time (e.g., an elapsed reaction time or a remaining reaction time) based on a measured optical characteristic value of a substance on the control line 25, and determining a measurement time at which to obtain an optical characteristic value of a substance on the test line 24 based on the estimated reaction time (e.g., the elapsed reaction time or the remaining reaction time).

The optical characteristic values of substances on the control line 25 and the test line 24 may be measured simultaneously or separately depending on the configuration of the detector 110 and/or the distance between the control line 25 and the test line 24.

For example, the controller 120 may control the detector 110, within a predetermined period of time measured from when the reactor 20 is inserted or the test is started, to measure optical characteristic values of substances on the control line 25 and/or the test line 24. The detector 110 may measure optical characteristic values of substances on the control line 25 and/or the test line 24 any number of times, and may store the results for use by the controller 120.

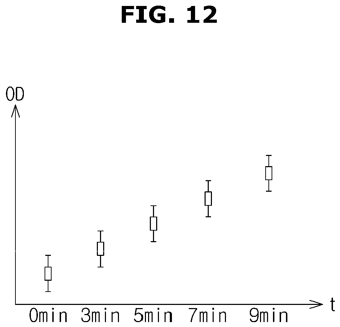

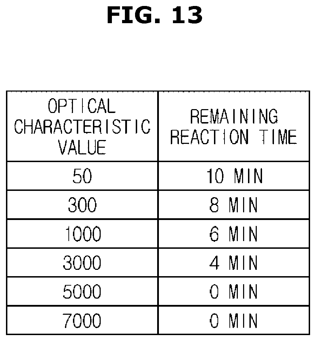

FIG. 12 is an exemplary graph representing optical characteristic values on a control line over the elapsed reaction time, and FIG. 13 shows an exemplary table of remaining reaction times matched with the respective optical characteristic values on a control line.

As shown in FIG. 12, the optical characteristic values, which are shown as OD values, of a substance on the control line 25 increase as the reaction time increases. The controller 120 may access predetermined reaction time information that maps (e.g., matches) optical characteristic values and respective reaction times. As used herein, predetermined reaction time information may refer to information that maps a known optical characteristic value and a known reaction time.

The controller 120 may estimate a reaction time based on a measured optical characteristic value and the predetermined reaction time information. For example, the controller 120 may estimate an elapsed reaction time or a remaining reaction time by comparing predetermined optical characteristic values, that are mapped to predetermined reaction times, and the measured optical characteristic values.

The controller 120 may estimate an elapsed reaction time based on an optical characteristic value measured on the control line 25 and the predetermined reaction time information. For example, the controller 120 may determine an elapsed reaction time corresponding to the optical characteristic value measured on the control line 25 by comparing predetermined optical characteristic values, that are mapped with respective elapsed reaction times, and the optical characteristic values measured on the control line 25. The elapsed reaction time corresponding to the optical characteristic value measured on the control line 25 may be estimated as the currently elapsed reaction time.

The controller 120 may determine a measurement time at which to measure an optical characteristic value of a substance on the test line 24 based on the estimated elapsed reaction time. For example, the controller 120 may determine a remaining reaction time based on the estimated elapsed reaction time, and determine the measurement time at which to measure an optical characteristic value of a substance on the test line 24 based on the remaining reaction time. For example, assuming that the estimated elapsed reaction time is three minutes and the color development completion time for detecting a target substance is ten minutes, then the controller 120 may determine that the measurement time is seven minutes measured from the current time (e.g., because 10 minutes - 3 minutes = 7 minutes).

As shown in FIG. 13, the predetermined reaction time information may map optical characteristic values and remaining reaction times. For example, and as shown in FIG. 13, the predetermined reaction time information may map a known optical characteristic value (e.g., 50) with a known remaining reaction time (e.g., 10 minutes). In this way, the controller 120 may compare a measured optical characteristic value (e.g., 50) and the predetermined reaction time information, and estimate a remaining reaction time (e.g., 10 minutes) based on the comparison. Put another way, the controller 120 may obtain a measured optical characteristic value, identify a known optical characteristic value that corresponds (e.g., is equal to) the measured optical characteristic value, identify a known remaining reaction time that is mapped to the known optical characteristic value, and estimate a remaining reaction time based on the known remaining reaction time.

The controller 120 may estimate a remaining reaction time based on an optical characteristic value measured on the control line 25. For example, the controller 120 may determine a remaining reaction time corresponding to the optical characteristic value measured on the control line 25 by comparing predetermined optical characteristic values and the optical characteristic value measured on the control line 25. The remaining reaction time corresponding to the optical characteristic value measured on the control line 25 may be estimated as the remaining reaction time.

The controller 120 may determine a measurement time at which to measure an optical characteristic value on the test line 24 based on the estimated remaining reaction time. For example, the controller 120 may determine that the measurement time is a time at which the remaining reaction time lapses. As an example, assume that the controller 120 determines, at a time point T, an estimated remaining reaction time of ten minutes. In this case, the controller 120 may determine the measurement time to be T+10 minutes. Put another way, the measurement time may be a time point at which the remaining reaction time lapses.

In some cases, the controller 120 may obtain the predetermined reaction time information from the reactor 20. The reactor 20 may include a mechanism that permits the controller 120 to obtain the predetermined reaction time information. For example, the reactor 20 may include a bar code (e.g., a one-dimensional bar code, a two-dimensional bar code, etc.), a quick response code, a near-field communication (NFC) chip, a radio frequency identification (RFID) tag, and/or the like, that permits the controller 120 to obtain the predetermined reaction time information. The controller 120 may obtain the predetermined reaction time information from the reactor 20 via the mechanism and information stored by the reactor 20. Alternatively, the controller 120 may obtain the predetermined reaction time information from another device via the mechanism of the reactor 20. For example, the controller 120 may provide a request to another device after scanning the mechanism of the reactor 20.

In addition to the predetermined reaction time information, the reactor 20 may permit the controller 120 to obtain information associated with the date of manufacture of the reactor 20, an expiration date of the predetermined reaction time information, information about a reaction occurring on the reactor 20, information about a wavelength used to measure the optical characteristic value on the test line 24 and the control line 25, a first reference value for the optical characteristic value on the control line 25, a second reference value for the optical characteristic value on the test line 24, a saturation value of the optical characteristic value, and/or the like.

Depending on the type of the mechanism of the reactor 20 (e.g., bar code, NFC chip, RFID tag, etc.), the mechanism may be read by the detector 110, or an extra reader, such as an RFID reader, an NFC reader, etc., that is equipped on the testing apparatus 100.

Once the reactor 20 is inserted into the testing apparatus 100, the detector 110 or the extra reader may read the mechanism to obtain the predetermined reaction time information, and the predetermined reaction time information may be temporarily or non-temporarily stored in the storage 130.

The controller 120 may determine a measurement time at which to measure an optical characteristic value of a substance on the test line 24 based on the predetermined reaction time information stored in the storage 130.

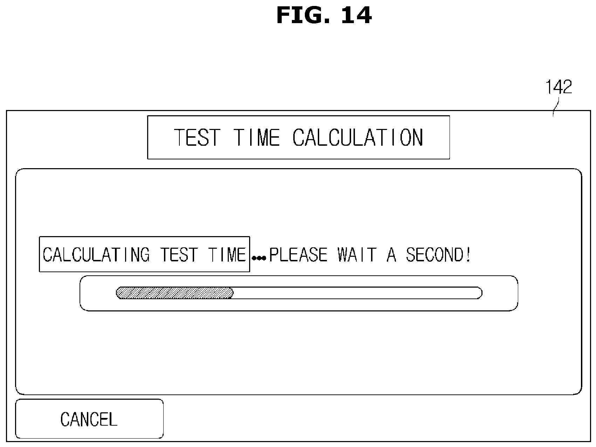

FIGS. 14, 15 and 16 show exemplary screens displayed on a display of a testing apparatus, according to an embodiment.

When the reactor 20 is inserted into the testing apparatus 100 and a test is started, the controller 120 may calculate a testing time. As used herein, the testing time may refer to an amount of time that remains until the test is finished. In other words, the testing time may correspond to a remaining reaction time. Testing on the reactor 20 may be started when the user manipulates the input interface 141 to enter a test start command or when the reactor 20 is inserted into the testing apparatus 100.

As shown in FIG. 14, the display 142 may display a screen providing information that identifies that the test time is currently being calculated while the controller 120 calculates the remaining reaction time.

As described above, when the test is started, the detector 110 or the extra reader may obtain the predetermined reaction time information via the reactor 20, and the predetermined reaction time information may be stored in the storage 130.

To calculate the remaining reaction time, the detector 110 may measure an optical characteristic value that represents a degree of color development of a substance on the control line 25 of the reactor 20, and determine the reaction time (e.g., the remaining reaction time or the elapsed reaction time) corresponding to the measured optical characteristic value by comparing the measured optical characteristic value with the predetermined reaction time information stored in the storage 130. The determined reaction time (e.g., the remaining reaction time or the elapsed reaction time) may be an estimate of the remaining reaction time or the elapsed reaction time.

In the situation where the controller 120 determines (e.g., estimates) an elapsed reaction time, the controller 120 may calculate the remaining reaction time based on an expected reaction completion time and the estimated elapsed reaction time. The expected reaction completion time may be obtained from the reactor 20.

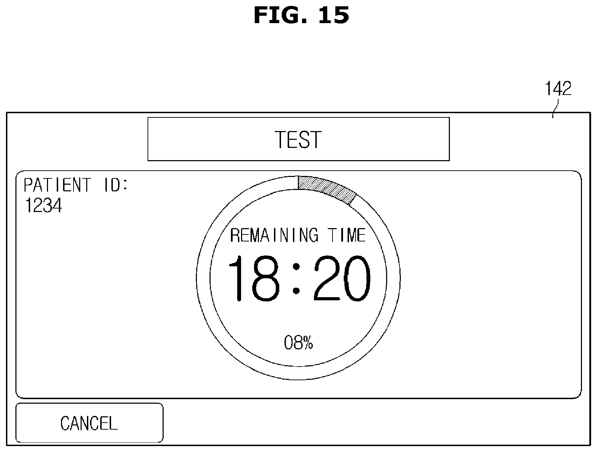

As shown in FIG. 15, the display 142 may display the remaining reaction time in situations where the controller 120 determines the remaining reaction time. Further, the display 142 may update a display of the remaining reaction time as the testing elapses. In this way, a user may be apprised of a status of the testing and/or the estimated remaining reaction time.

The controller 120 may control the detector 110 to cause the detector 110 to measure an optical characteristic value that represents a degree of color development of a substance on the test line 24 at a point in time when the calculated remaining reaction time lapses.

The controller 120 may determine the presence of a target substance or the concentration of the target substance based on the optical characteristic value measured on the test line 24, and determine a validity of the test based on the optical characteristic value measured on the control line 25. By utilizing the measurement time, some embodiments herein improve accuracy of testing and/or the reliability of test results.

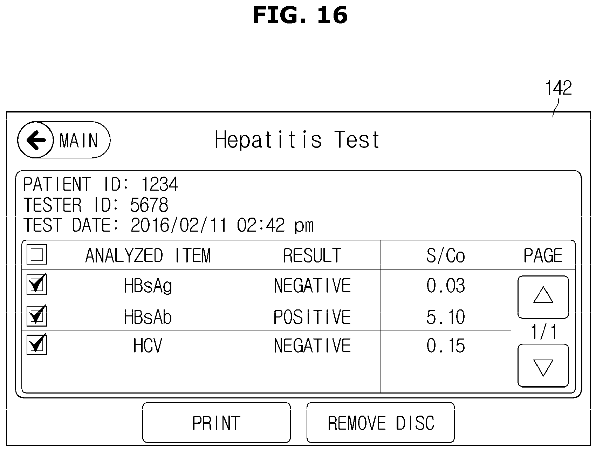

As shown in FIG. 16, the controller 120 may cause the display 142 to display information identifying the presence (or absence) of the target substance, a concentration of the target substance, whether the test result is positive, negative, invalid, etc., patient information, tester information, temporal information, and/or the like.

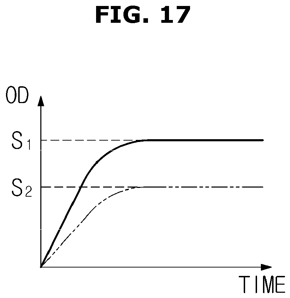

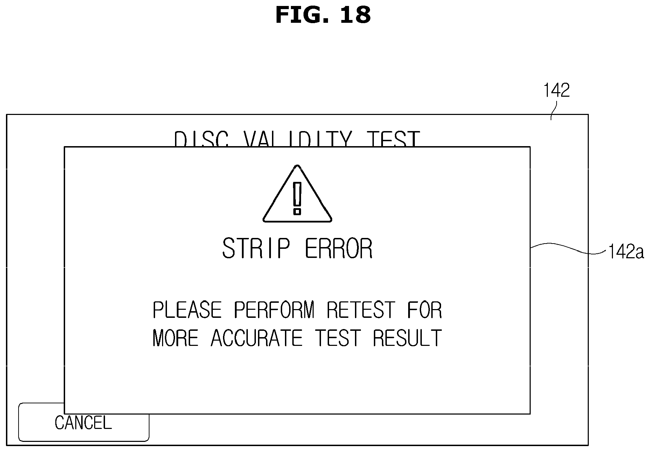

FIG. 17 is an exemplary graph representing saturation values of optical characteristic values on a control line, and FIG. 18 shows an exemplary warning screen displayed when a flow error occurs on a reactor.

As shown in FIG. 17, the optical characteristic value increases as time elapses until the optical characteristic value reaches a saturation value (e.g., becomes saturated). In other cases, optical characteristic values may decrease over time.

In situations where a sample is moved in a relatively uninhibited manner on the reactor 20 by capillary force, the optical characteristic value of the substance formed on the control line 25 reaches a certain reference value S1. In this case, the certain reference value S1 may correspond to a predetermined saturation value. The predetermined saturation value S1 may be determined based on the type of color reaction on the control line 25, and may be obtained from the reactor 20 along with the predetermined reaction time information.

When the detector 110 sends the optical characteristic value measured on the control line 25 to the controller 120, the controller 120 may determine whether the measured optical characteristic value on the control line is saturated. For example, if the controller 120 determines that there is substantially no change in the optical characteristic value on the control line 25 measured across a predetermined period of time or if the change is less than a predetermined reference change, the controller 120 may determine that the optical characteristic value on the control line is saturated.

If a measured saturation value S2 is less than the predetermined saturation value S1, then the controller 120 may determine that the sample has failed to flow effectively across the reactor 20. In other words, if the measured saturation value S2 is less than the normal saturation value S1, the controller 120 may determine that an error in the flow has occurred.

As shown in FIG. 18, and in the situation where the controller 120 determines that an error has occurred, the controller 120 may control the display 142 to output an error warning screen 142a. In this way, a user may be apprised of the error, and may perform a retest of the sample.

While a visual warning is shown in FIG. 18, other embodiments include audible warnings and/or other types of warnings in addition to, or in lieu of, the visual warning.

A control method of a testing apparatus in accordance with an embodiment will now be described. The control method of a testing apparatus may be employed by the testing apparatus 100. What has been described above in association with reference to FIGS. 1 to 18 may also be applied to the control method of the testing apparatus.

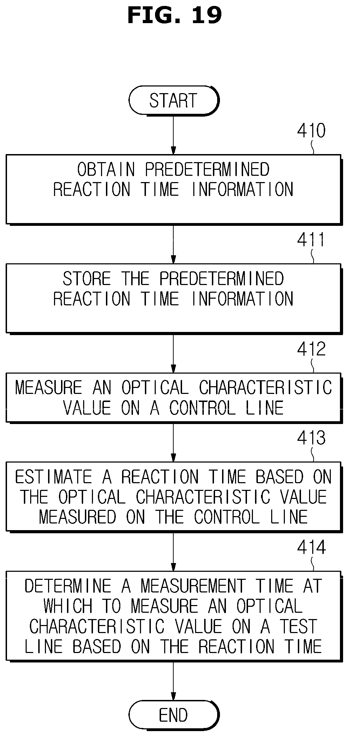

FIG. 19 is a flowchart illustrating a control method of a testing apparatus, according to an embodiment.

As shown in FIG. 19, the method may include obtaining predetermined reaction time information (410). For example, the controller 120 of the testing apparatus 100 may obtain predetermined reaction time information based on the reactor 20 being inserted into the testing apparatus 100. The reaction time refers to an elapsed reaction time or a remaining reaction time. The predetermined reaction time information may include a table that stores predetermined optical characteristic values that are mapped to respective reaction times for the same reaction. For example, the predetermined reaction time information may be obtained from the reactor 20 (e.g., via scanning a bar code, or utilizing another type of mechanism).

As further shown in FIG. 19, the method may include storing the predetermined reaction time information (411). For example, the testing apparatus 100 may store the predetermined reaction time information in storage 130.

As further shown in FIG. 19, the method may include measuring an optical characteristic value on a control line (412). For example, the controller 120 of testing apparatus 100 may cause the detector 110 to measure an optical characteristic value of a substance on a control line 25 of the reactor 20. The optical characteristic value measured on the control line may represent a degree of color development of a substance on the control line 25. The controller 120 may control the detector 110 to measure the optical characteristic value of a substance on the control line 25. For example, once the light source of the detector 110 generates light of a particular wavelength band and irradiates the light onto the control line 25, the receiver of the detector 110 may measure an optical characteristic value of a substance on the control line 25 by receiving light reflecting from the control line 25. Alternatively, the detector 110 may be implemented with a camera and may measure the optical characteristic value of a substance on the control line 25 by capturing an image of the control line 25.

As further shown in FIG. 19, the method may include estimating a reaction time based on the optical characteristic value measured on the control line (413). For example, the controller 120 of the testing apparatus 100 may estimate a reaction time based on the optical characteristic value measured on the control line 25 and the predetermined reaction time information. The reaction time may correspond to an elapsed reaction time or a remaining reaction time, and may be estimated by comparing predetermined optical characteristic values and the measured optical characteristic value. The controller 120 may estimate an elapsed reaction time based on the optical characteristic value measured on the control line 25. Alternatively, the controller 120 may estimate a remaining reaction time based on the optical characteristic value measured on the control line 25.

As further shown in FIG. 19, the method may include determining a measurement time at which to measure an optical characteristic value on a test line based on the reaction time (414). For example, the controller 120 of the testing apparatus 100 may determine a measurement time based on the reaction time, and cause the detector 110 to measure an optical characteristic value of a substance on the test line 24 based on the measurement time. The controller 120 may determine the measurement time based on the estimated elapsed reaction time. For example, the controller 120 may calculate a remaining reaction time based on the estimated elapsed reaction time and a known completion time, and determine the measurement time based on the remaining reaction time. Alternatively, the controller 120 may determine the measurement time based on the estimated remaining reaction time. Specifically, the controller 120 may determine the measurement time at which to measure the optical characteristic value of a substance on the test line 24 to be a point in time at which the estimated remaining reaction time lapses from the current point in time.

When measuring the optical characteristic value on the test line 24 at the determined measurement time, the controller 120 may determine the presence of a target substance by comparing the optical characteristic value measured on the test line 24 with the predetermined second reference value, and display the results of the determination on the display 142. The controller 120 may calculate the concentration of the target substance using a predetermined calibration curve.

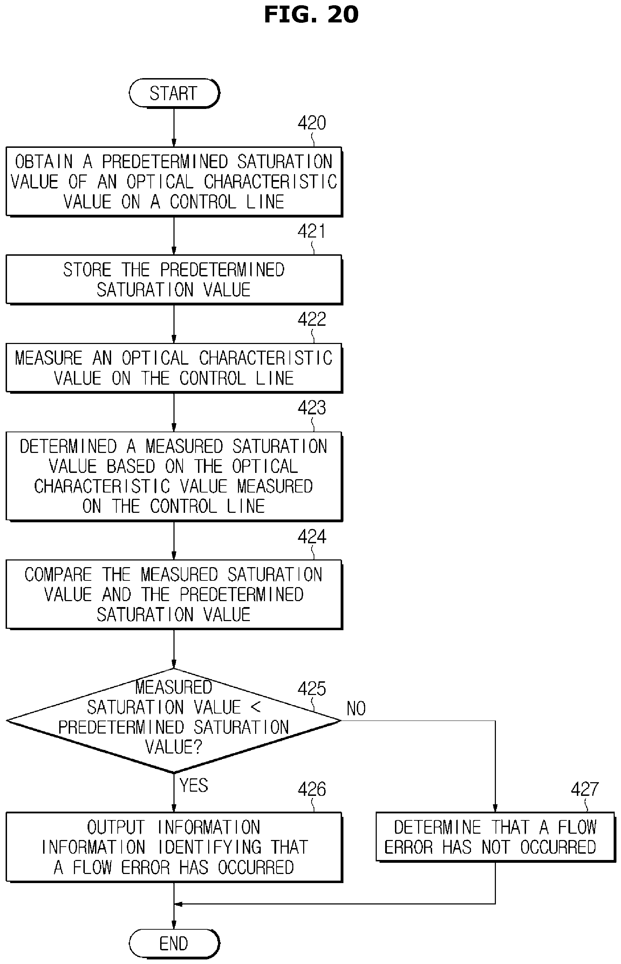

FIG. 20 is a flowchart illustrating a method of detecting a flow error in association with a control method of a testing apparatus.

As shown in FIG. 20, the method may include obtaining a predetermined saturation value of an optical characteristic value on a control line (420). For example, the controller 120 of the testing apparatus 100 may obtain a predetermined saturation value. The predetermined saturation value refers to a saturation value that appears on the control line 25 when the sample flows across the reactor 20 by capillary force substantially without error. The testing apparatus 100 may obtain the predetermined saturation value from the reactor 20, such as via scanning a bar code of the reactor 20 and/or the like.

As further shown in FIG. 20, the method may include storing the predetermined saturation value (421). For example, the controller 120 of the testing apparatus 100 may store the predetermined saturation value in storage 130, such as in volatile or non-volatile memory.

As further shown in FIG. 20, the method may include measuring an optical characteristic value on the control line (422). For example, the controller 120 of the testing apparatus 100 may cause the detector 110 to measure a set of optical characteristic values of a substance on the control line 25. In other words, the testing apparatus 100 may measure optical characteristic values of a substance at predetermined intervals associated with a time frame.

As further shown in FIG. 20, the method may include determining a measured saturation value based on the optical characteristic value measured on the control line (423). For example, the controller 120 of the testing apparatus 100 may determine a measured saturation value based on a set of optical characteristic values measured on the control line 25. The testing apparatus 100 may determine that a change in optical characteristic values measured on the control line 25 is less than a predetermined change value, and determine a saturation value based on the change being less than the predetermined change value. In other words, the testing apparatus 100 may determine that an optical characteristic value has reached a saturation point in the situation where the optical characteristic values measured on the control line 25 are not changing across respective measurements or are changing by less than a threshold amount across respective measurements. In this way, the controller 120 of the testing apparatus 100 may determine a measured saturation value based on the optical characteristic value reaching the saturation point.

As further shown in FIG. 20, the method may include comparing the measured saturation value and the predetermined saturation value (424). For example, the controller 120 of the testing apparatus 100 may compare the measured saturation value and the predetermined saturation value to determine whether a flow error has occurred.

As further shown in FIG. 20, the method may include determining whether the measured saturation value is less than the predetermined saturation value (425). For example, the controller 120 of the testing apparatus 100 may determine whether the measured saturation value is less than the predetermined saturation value based on comparing the measured saturation value and the predetermined saturation value.

As further shown in FIG. 20, if the measured saturation value is less than the predetermined saturation value (425 - YES), then the method may include outputting information identifying that a flow error has occurred (426). For example, the controller 120 of the testing apparatus 100 may output information via display 142 that identifies that a flow error has occurred. The information identifying that the flow error has occurred may including visual information, audio information, and/or a combination thereof. In this way, a user may identify that a flow error has occurred, and perform a retest and/or the like. Additionally, in this way, the reliability and/or accuracy of testing may be improved.

As further shown in FIG. 20, if the measured saturation value is not less than the predetermined saturation value (425 - NO), then the method may include determining that a flow error has not occurred (427). For example, the controller 120 of the testing apparatus 100 may determine that a flow error has not occurred based on the measured saturation value being greater than or equal to the predetermined saturation value. The controller 120 of the testing apparatus 100 may cause display 142 to provide information for display that identifies that the testing has occurred substantially without error. Additionally, or alternatively, the controller 120 of the testing apparatus 100 may cause a component of testing apparatus 100 to output audible information identifying that a flow error has not occurred.

In this way, the controller 120 of the testing apparatus 100 may detect a flow error based on a comparison between a measured saturation value and a predetermined saturation value. In other words, a measured saturation value that is less than a predetermined saturation value may be indicative of a flow error associated with the sample.

The embodiments of the disclosure increase the reliability and/or accuracy of testing by accounting for a time difference measured from when a sample is applied to a reactor and when the testing is started. For example, the embodiments permit measured optical characteristic values to be compared with predetermined optical characteristic values that are mapped to reaction times. In this way, the embodiments permit the testing apparatus to estimate a reaction time (e.g., an elapsed reaction time or a remaining reaction time) based on the comparison, and determine a measurement time at which to measure an optical characteristic value based on the reaction time.

In addition, some embodiments permit the testing apparatus to determine that an error has occurred in association with the testing, and output information identifying that the error has occurred. In this way, some embodiments improve reliability and/or accuracy of testing by notifying a user of situations that may result in an inaccurate test.

According to embodiments, a testing apparatus and control method thereof may improve reliability of a test result by estimating the elapsed reaction time based on a degree of color development on a control line of a reactor in which a reaction between a sample and a reagent occurs and determining a measurement time at which to measure a degree of color development on a test line based on the estimated elapsed reaction time.

Further, the testing apparatus and control method thereof may improve reliability of test results by determining and informing the user that an error in a flow of a sample has occurred when the saturation value of an optical characteristic value measured on a control line varies from a predetermined reference value.

Although a few embodiments have been shown and described herein, it should be appreciated by those skilled in the art that changes may be made to these embodiments without departing from the principles and spirit of the disclosure, the scope of which is defined in the appended claims and their equivalents.

## Claims

1. A testing apparatus comprising:
a detector configured to measure optical characteristic values on a control line and a test line formed on a reactor; and
a controller configured to:
obtain an optical characteristic value measured, by the detector, on the control line;
determine a reaction time corresponding to the optical characteristic value measured on the control line based on predetermined reaction time information; and
determine a measurement time at which the detector is to measure another optical characteristic value on the test line based on the reaction time that is determined.

2. The testing apparatus of claim 1, wherein the reaction time comprises an elapsed reaction time or a remaining reaction time.

3. The testing apparatus of claim 1, wherein the predetermined reaction time information comprises information that matches predetermined optical characteristic values with respective reaction times.

4. The testing apparatus of claim 2, wherein the controller is further configured to determine the measurement time based on the elapsed reaction time and an expected reaction completion time based on the reaction time comprising the elapsed reaction time.

5. The testing apparatus of claim 2, wherein the controller is further configured to determine the measurement time based on the remaining reaction time and a current time based on the reaction time comprising the remaining reaction time.

6. The testing apparatus of claim 1, further comprising:
a storage configured to store the predetermined reaction time information when the reaction time information is obtained by the testing apparatus using information associated with the reactor.

7. The testing apparatus of claim 2, further comprising:
a display configured to display the reaction time.

8. The testing apparatus of claim 1, wherein the controller is further configured to compare a saturation value of the optical characteristic value measured on the control line with a predetermined saturation value and determine whether an error has occurred based on a result of the comparison.

9. The testing apparatus of claim 8, wherein the controller is further configured to output information identifying that the error has occurred based on the saturation value of the optical characteristic value measured on the control line being less than the predetermined saturation value.

10. The testing apparatus of claim 8, wherein the controller is further configured to determine that the optical characteristic value measured on the control line is saturated based on a change in the optical characteristic value measured on the control line within a predetermined period of time being less than a predetermined reference change.

11. A control method of a testing apparatus, the control method comprising:
obtaining, by the testing apparatus, predetermined reaction time information for a control line;
measuring, by the testing apparatus, an optical characteristic value on the control line;
determining, by the testing apparatus, a reaction time corresponding to the optical characteristic value measured on the control line based on the predetermined reaction time information; and
determining, by the testing apparatus, a measurement time at which the testing apparatus is to measure another optical characteristic value on a test line based on the reaction time.

12. The control method of claim 11, wherein the reaction time comprises an elapsed reaction time or a remaining reaction time.

13. The control method of claim 11, wherein the predetermined reaction time information comprises information that maps predetermined optical characteristic values with respective reaction times.

14. The control method of claim 12, wherein the determining of the measurement time comprises determining the measurement time based on the elapsed reaction time and an expected reaction completion time based on the reaction time comprising the elapsed reaction time.

15. The control method of claim 12, wherein the determining of the measurement time comprises determining the measurement time based on the remaining reaction time and a current time based on the reaction time comprising the remaining reaction time.

## Amended claims

### Amended claims in accordance with Rule 137(2) EPC.

1. A testing apparatus (100) comprising:
a main body (102) configured to receive a reactor (20);
a detector (110) configured to measure optical characteristic values on a control line (25) and a test line (24) formed on the reactor (20); and
a controller (120) configured to:
obtain an optical characteristic value measured, by the detector (110), on the control line (25);
determine a reaction time corresponding to the optical characteristic value measured on the control line (25) based on predetermined reaction time information;
determine a measurement time at which the detector (110) is to measure another optical characteristic value on the test line (24) based on the reaction time that is determined;
compare a saturation value of the optical characteristic value measured on the control line (25) with a predetermined saturation value; and
determine whether an error has occurred based on a result of the comparison.

2. The testing apparatus (100) of claim 1, wherein the reaction time comprises an elapsed reaction time or a remaining reaction time.

3. The testing apparatus (100) of claim 1, wherein the predetermined reaction time information comprises information that matches predetermined optical characteristic values with respective reaction times.

4. The testing apparatus (100) of claim 2, wherein the controller (120) is further configured to determine the measurement time based on the elapsed reaction time and an expected reaction completion time based on the reaction time comprising the elapsed reaction time.

5. The testing apparatus (100) of claim 2, wherein the controller (120) is further configured to determine the measurement time based on the remaining reaction time and a current time based on the reaction time comprising the remaining reaction time.

6. The testing apparatus (100) of claim 1, further comprising:
a storage (130) configured to store the predetermined reaction time information when the reaction time information is obtained by the testing apparatus (100) using information associated with the reactor (20).

7. The testing apparatus (100) of claim 2, further comprising:
a display (142) configured to display the reaction time.

8. The testing apparatus (100) of claim 1, wherein the controller (120) is further configured to output information identifying that the error has occurred based on the saturation value of the optical characteristic value measured on the control line (25) being less than the predetermined saturation value.

9. The testing apparatus (100) of claim 1, wherein the controller (120) is further configured to determine that the optical characteristic value measured on the control line (25) is saturated based on a change in the optical characteristic value measured on the control line (25) within a predetermined period of time being less than a predetermined reference change.

10. A control method of a testing apparatus (100), the control method comprising:
obtaining, by the testing apparatus (100), predetermined reaction time information for a control line (25) of a reactor (20) being inserted into a main body (102) of the testing apparatus (100) from a storage (130) of the testing apparatus (100);
measuring, by the testing apparatus (100), an optical characteristic value on the control line (25);
determining, by the testing apparatus (100), a reaction time corresponding to the optical characteristic value measured on the control line (25) based on the predetermined reaction time information;
determining, by the testing apparatus (100), a measurement time at which the testing apparatus (100) is to measure another optical characteristic value on a test line (24) of the reactor (20) based on the reaction time;
comparing, by the testing apparatus (100), a saturation value of the optical characteristic value measured on the control line (25) with a predetermined saturation value; and
determining, by the testing apparatus (100), whether an error has occurred based on a result of the comparison.

11. The control method of claim 10, wherein the reaction time comprises an elapsed reaction time or a remaining reaction time.

12. The control method of claim 10, wherein the predetermined reaction time information comprises information that maps predetermined optical characteristic values with respective reaction times.

13. The control method of claim 11, wherein the determining of the measurement time comprises determining the measurement time based on the elapsed reaction time and an expected reaction completion time based on the reaction time comprising the elapsed reaction time.

14. The control method of claim 11, wherein the determining of the measurement time comprises determining the measurement time based on the remaining reaction time and a current time based on the reaction time comprising the remaining reaction time.
